# EUROPÄISCHE PATENTSCHRIFT

(11) **EP 1 937 765 B1**
(45) Veröffentlichungstag und Bekanntmachung des Hinweises auf die Patenterteilung: **17.06.2009**
(21) Anmeldenummer: 06777199.8
(22) Anmeldetag: 23.09.2006
(51) Int. Cl.: C08K 5/00, A61L 29/06, A61L 29/16

(54) **VERFAHREN ZUR HERSTELLUNG ANTIMIKROBIELLER KUNSTSTOFFZUSAMMENSETZUNGEN**
METHOD FOR PRODUCING ANTIMICROBIAL PLASTIC COMPOSITIONS
PROCEDE DE PRODUCTION DE COMPOSITIONS DE MATIERE PLASTIQUE ANTIMICROBIENNES

(30) Priorität: 06.10.2005 DE 102005048132
(43) Veröffentlichungstag der Anmeldung: 02.07.2008
(73) Patentinhaber: Bayer Innovation GmbH, 40225 Düsseldorf (DE)
(72) Erfinder: PUDLEINER, Heinz, 47800 Krefeld (DE); HYNER, Joachim, 40764 Langenfeld (DE)
(74) Vertreter: Hildebrand, Steven
(86) Internationale Anmeldenummer: PCT/EP2006/009266
(87) Internationale Veröffentlichungsnummer: WO 2007/039157

(56) Entgegenhaltungen:
- EP-A2- 0 470 443
- DE-A1- 2 708 331
- DE-A1- 19 852 192
- DE-C1- 19 812 160
- US-B1- 6 641 831
- DATABASE WPI Week 199634 Derwent Publications Ltd., London, GB; AN 1996-339316 XP002410422 & JP 08 157641 A (UNITIKA LTD) 18. Juni 1996 (1996-06-18) in der Anmeldung erwähnt
- DATABASE CAPLUS [Online] CHEMICAL ABSTRACTS SERVICE, COLUMBUS, OHIO, US; 26. Januar 2006 (2006-01-26), KOMA, HIROKI ET AL: "Antibacterial and antifungal resin compositions containing bispyridinium compounds" XP002410418 gefunden im STN Database accession no. 2006:73712 -& JP 2006 022217 A (TAMA KAGAKU KOGYO CORPORATION, JAPAN) 26. Januar 2006 (2006-01-26)
- J.C.SANTAMARINA, K.A.KLEIN, Y.H.WANG, E.PRENCKE: "Specific surface:determination and relevance" CAN. GEOTECH. J., [Online] Bd. 39, 2002, Seiten 233-241, XP002410626 Gefunden im Internet: URL:http://www.ce.gatech.edu/~carlos/labor atory/tool/specificsurface/Paper%20on%20Sp ecific%20Surface.pdf>

## Beschreibung

Die vorliegende Erfindung betrifft ein Verfahren zur Herstellung antimikrobieller Kunststoffzusammensetzungen aus einem Termoplasten, besonders thermoplastischen Elastomer, und mindestens einem pulverförmigen antimikrobiellen Wirkstoff, speziell aus der Gruppe der Bis-(4-amino-1-pyridinium)-alkane, und die Verwendung dieser Kunststoffzusammensetzung für Katheter und andere medizintechnische Produkte.

Zahlreiche Studien haben ergeben, dass Koagulase negative Staphylococcen, der transiente Keim Staphylococcus aureus, Staphylococcus epidermis und verschiedene Candida Spezies die Hauptverursacher von Katheter assoziierten Infektionen sind. Diese ubiquitär auf der Haut vorhandenen Mikroorganismen durchdringen bei der Applikation des Katheters die physiologische Hautbarriere und gelangen so in den subkutanen Bereich und letztendlich in die Blutbahn. Die Adhäsion der Bakterien auf der Kunststoffoberfläche wird als essentieller Schritt bei der Pathogenese von Fremdkörperinfektionen betrachtet. Nach der Adhäsion der Hautkeime an der Polymeroberfläche beginnt die metabolisch aktive Proliferation der Bakterien mit der Besiedlung des Polymers. Damit einher geht die Produktion eines Biofilms durch bakterielle Exkretion von extrazellulärer Glykocalix.

Durch prä-, peri- oder postoperative Massnahmen (z. B. hygienische Maßnahmen etc.) kann diese Problematik nur teilweise gelöst werden. Eine rationale Strategie zur Prävention von Polymer assoziierten Infektionen besteht in der Modifizierung der verwendeten polymeren Materialien. Ziel dieser Modifizierung muss die Hemmung der Bakterienadhäsion bzw. der Proliferation bereits adhärierter Bakterien sein, um somit kausal Fremdkörperinfektionen zu vermeiden. Dies kann z. B. durch Inkorporierung eines geeigneten Chemotherapeutikums in die Polymermatrix (z. B. Antimikrobiotika wie Antibiotika und Antiseptika) gelingen, vorausgesetzt, dass der eingearbeitete Wirkstoff auch aus der Polymermatrix herausdiffundieren kann. In diesem Fall kann die Freisetzung des antimikrobiellen Wirkstoffes auf einen längeren. Zeitraum ausgedehnt werden, damit für einen entsprechend längeren Zeitraum die Mikroben- respektive Bakterienadhäsion bzw. deren Proliferation auf dem Polymer verhindert wird.

Methoden zur Herstellung antimikrobiell ausgerüsteter Polymere sind bereits bekannt. Hierbei werden die Mikrobizide auf die Oberfläche oder eine Oberflächenschicht aufgebracht oder in den polymeren Werkstoff eingebracht. Für die insbesondere für medizinische Anwendungen verwandten thermoplastischen Polyurethane sind folgende Techniken beschrieben:
a) Adsorption auf der Polymeroberfläche (passiv oder via Surfactants)
b) Einbringen in eine Polymerbeschichtung, die auf der Oberfläche eines Formkörpers appliziert wird
c) Inkorporierung in die bulk-Phase des polymeren Trägermaterials
d) Kovalente Bindung an der Polymeroberfäche
e) Mischen mit einer Polyurethan bildenden Komponente vor der Reaktion zum fertigen Polymer

In der Folge soll nur der Stand der Technik, der sich mit der Inkorporierung von Wirkstoffen in die bulk-Phase des polymeren Trägermaterials beschäftigt, näher beleuchtet werden.

Das US P 5,281,677 beschreibt Blends aus TPU, welche bevorzugt zur Herstellung von mehrlumigen vaskulären Katheter eingesetzt werden. Erwähnt wird, dass die Formkörper auch einen antimikrobiellen Wirkstoff enthalten können, der in einem der Polyurethane vor dem Schmelzprozess gleichgewichtsverteilt (bulk-distributed) sein kann.

Das US P 6,120,790 beschreibt thermoplastische Harze, die antimikrobielle oder fungistatische Wirkstoffe enthalten, wobei das Polymer eine Polyetherkette als Baustein enthält. Als Wirkstoffe kämen unter organischen Verbindungen auch Pyridine infrage, ohne dass diese beispielsweise spezifiziert werden.

Die EP 927 222 B1 beschreibt die Einbringung von antithrombisch oder antibiotisch wirksamer Substanzen in die Reaktionsmischung zur Herstellung eines TPUs.

US P 5,906,825 beschreibt Polymere, darunter auch Polyurethane, in denen Biozide respektive Antimikrobiotika (konkret beschrieben sind ausschließlich Pflanzeninhaltsstoffe) in solcher Menge dispergiert sind, dass das Wachstum von Mikroorganismen unterbunden wird, die mit dem Polymer in Berührung kommen. Dieses kann durch Zusatz von einem Agenz optimiert werden, welches die Wanderung und/oder Freisetzung des Biozids reguliert. Erwähnt werden Naturstoffe wie z.B. Vitamin E. Anwendungsschwerpunkt sind Lebensmittelverpackungen.

Die JP 08-157641 beschreibt ein Verfahren zur Herstellung von antimikrobiellen Materialien durch Schmelzkneten eines Polymers mit einer spezifischen Oberfläche von größer gleich 17 cm²/g, darunter u. a. Polyurethan, mit einem pulverförmigen Wirkstoffes, Bevorzugt Chlorhexidin.

Die CN 1528470 A beschreibt ein Verfahren zur Herstellung eines medizinischen antiinfektiösen Einführungshilieschlauches für Katheter aus Polyurethan, wobei ein als Muttermaterial bezeichneter Masterbatch, welcher das Antimikrobiotikum enthält, mit dem PU-Rohmaterial gemischt und zum Formkörper extrudiert wird.

Allen erwähnten Verfahren ist gemeinsam, dass mindestens ein antimikrobieller Wirkstoffe in die Schmelze des polymeren Trägermaterials eingebracht wird, und die zeitlich begrenzte Langzeitwirkung der antimikrobiellen Ausrüstung der Formkörper aus polymerem Material, insbesondere von Medizinprodukten beim Einsatz am bzw. im Patienten, optimiert wird. Dieses sowie die Vermeidung der Gefahr einer mikrobiellen Erstinfektion des Formkörpers selber oder von Mensch und Tier durch den Formkörper werden hierbei aber nicht gleichzeitig Beides befriedigend gewährleistet.

Die hier gemeinten Medizinprodukte werden überwiegend intracorporal angewandt. Katheter beispielsweise durchdringen die Körperoberfläche für die gesamte Zeit der Anwendung und stellen deshalb ein besonders hohes Risiko für mikrobielle Infektionen dar, wie schon weiter oben dargelegt wurde. Die Gefahr von Erstinfektionen beim Einbringen der Medizinprodukte in den Körper durch mikrobielle Kontamination wird durch die bekannten antimikrobiellen Ausrüstungen noch nicht ausreichend reduziert.

Die üblichen Wirkstoffe liegen in der Regel als feine Pulver vor. Für die gewünschte Wirkung auf der Kunststoffoberfläche werden nur geringe Mengen der Wirkstoffe im Kunststoff benötigt. Dazu war es notwendig, dass kleine Mengen der antimikrobiell wirksamen Substanzen genau dosiert werden und dabei in allen Bereichen des medizinschen Produktes in gleicher Konzentration fein verteilt vorliegen.

Aufgabe der Erfindung war es, ein Verfahren für die Herstellung antimikrobiell ausgerüsteter Kunststoffe, insbesondere für medizinische Artikel wie Katheter, bereitzustellen, in denen mindestens ein antimikrobieller Wirkstoff sehr fein verteilt vorliegt, die für einem längeren Zeitraum (>4 Wochen) effizient eine Oberflächenbesiedlung durch Keime verhindern und über den Zeitraum von 15 Tagen kontinuierlich Wirkstoff abgeben.

Zur Lösung dieser Aufgabe wurde nun gefunden, dass sich dies erreichen lässt, wenn anstelle der üblichen Dosierung des reinen Wirkstoffes als Pulver in die Schmelze des Kunststoffs ein Gemisch des Wirkstoff mit Kunststoff-Pulver eingesetzt wird.

Es wurde durch dieses Verfahren gewährleistet, dass Formkörper eingesetzt werden, die über einen längeren Zeitraum über eine die Besiedelung mit Keimen unterbindende Konzentration an der Kunststoffoberfläche verfügen.

Vorzugsweise sind diese Kunststoffzusammensetzungen antimikrobiell, dadurch gekennzeichnet, dass die Konzentration des Wirkstoffes ausreichend ist, damit über einen längeren Zeitraum die Besiedelung mit unerwünschten Keimen unterbunden, zumindest signifikant reduziert wird. Dieser Zeitraum beträgt bevorzugt mindestens 2 Wochen, besonders bevorzugt mehr als 4 Wochen. Unter unerwünschten Keimen werden jeweils bestimmte Bakterien, Viren und Pilze verstanden.

Ein weiterer Gegenstand dieser Erfindung ist die Herstellung der erfindungsgemäßen Kunststoffzusammensetzung. Die erfindungsgemäßen Kunststoffzusammensetzungen werden bevorzugt durch thermoplastische Verarbeitung hergestellt und weiterverarbeitet.

Grundsätzlich sind folgende Wirkstoffe, soweit sie antimikrobielle Eigenschaften haben, geeignet: Ansamycinderivate (Rifamycin, Rifapentin), vorzugsweise sind antimikrobielle Substanzen genannt, welche auch klinischen Nutzen gefunden haben für sogenannte "Difficult to Treat Infections". Denkbar sind grundsätzlich alle antimikrobiell wirksamen Gruppen wie lipophile Vertreter aus der Gruppe der Aminoglykoside, aus der Gruppe der Cephalosporine und darauf bezogenen Betalactame, des Chloramphenicols, Lincosamiden, Makroliden, Penicillinen, Chino-Ionen, Sulfonamiden, Tetracyclinen, ausgenommen der Kombination Tetracyclin-Minocyclin. Vorzugsweise lipophile Antibiotika sind Benzathin, Phenoxymethylpenicillin, Chloramphenicol, Chlortetracyclin, Ciprofloxacin-Betain, Ciprofloxacin, Clarithromycin, Clindamycin-Palmitathydrochlorid, Trimethoprim, Erythromycin-2-Acetat, -Stearat; Erythromycin-Estolat, Erythromycin-Ethylsüccinat-, Erythromycin-Glutamat, -Laktopropionat, -Stearat, Fusidinsäure, vorzugsweise freie Fusidinsäure, Gramicidin, Mupirocin, lipophile Vertreter der Imidazolreihe wie Econazol, Itraconazol, Clotrimazol und andere, Pristinamycin, Rifabutin, Rifapentin, Rifampicin, Silbersulfadiazin.

Als besonders bevorzugte Wirkstoffe kommen grundsätzlich alle die in der DE 27 08 331 C2 in den Patentansprüchen 1 bis 4 auf S. 28 definierten Wirkstoffe in Frage. Bevorzugt werden die Verbindungen aus den Beispielen 1-82 (S. 5 bis S. 18 Z. 19), besonders bevorzugt wird Octenidin, sein Hydrochlorid oder ganz besonders bevorzugt das Dihydrochlorid 1,1'-(1,10-Decandiyl)bis[4-(octylamino)-pyridinium]-dichlorid eingesetzt.

Diese als Bis-[4-(substituierten-amino)-1-pyridinium]-alkane bezeichneten Wirkstoffe sind durch die allgemeinen Formeln (I) und (II) definiert 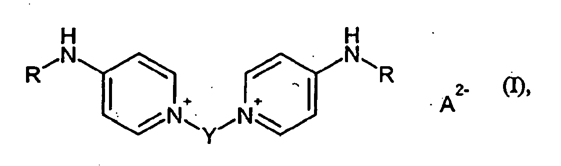 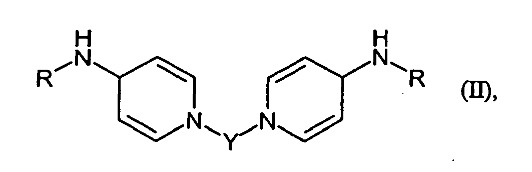 in welchen
- Y: für eine Alkylengruppe mit 4 bis 18 Kohlenstoffatomen steht,
- R: für C₆-C₁₈-Alkyl, C₅-C₇-Cycloalkyl oder durch ein Halogenatom substituiertes Phenyl steht und
- A: für zwei einwertige oder ein zweiwertiges Anion steht.
- Y: steht bevorzugt für 1,10-Decylen oder 1,12-Dodecylen, besonders bevorzugt für 1,12-Dodecylen.
- R: steht bevorzugt für n-Hexyl, n-Heptyl oder n-Octyl, besonders bevorzugt für n-Octyl.
- A: steht beispielsweise für ein Sulfat-, je 2 Fluorid-, Chlorid-, Bromid-, Jodid-, oder Methansulfonat-Ionen, bevorzugt für je 2 Fluorid-, Chlorid-, Bromid-, besonders bevorzugt für 2 Chlorid-Ionen.

Die Formel (II) bezeichnet die korrespondierenden freien Basen, welche durch Neutralisation aus den Salzen der Formel (I) nach den üblichen Methoden der organischen Chemie hergestellt werden können. Die Salze der Formel (I) werden in der Literatur häufig auch in Form der Formel (III)

Formel (II) x H₂A (III),

worin "Formel (II)" und A die oben angegebenen Bedeutungen haben, dargestellt. Naturgemäß ist eine chemische Formel nur ein vereinfachte Darstellung der Realität. In diesem Falle sind es Tautomere, für die es keinen Anhaltspunkt gibt, dass diese unter gebräuchlichen Bedingungen und Temperaturen unterscheidbar sind. Für Octenidin-dihydrochlorid gibt es dennoch je 2 Chemical Abstracts Registry Nummern und 2 Nummern im Europäischen Altstoffinventar. Für die Erfindung soll es nicht relevant sein, ob Verbindungen der Formel (I) oder der Formel (III) eingesetzt werden oder in welcher Form diese in der Polymerzusammensetzung vorliegen. Bevorzugt werden Salze der Formel (I) bzw. (III) eingesetzt.

Üblicherweise hat der Wirkstoff eine mittlere Teilchengröße d₅₀ von = 0,5 bis 20 µm, bevorzugt von 1 bis 10 µm.

Als Kunststoffe eignen sich grundsätzlich alle Thermoplaste, soweit ihre Verarbeitungstemperatur nicht zu Deaktivierung des eingesetzten Wirkstoffs führt, besonders jedoch thermoplastische Elastomere (TPE). TPE sind Werkstoffe, die elastomere Phasen in thermoplastisch verarbeitbaren Polymeren entweder physikalisch eingemischt oder chemisch eingebunden enthalten. Man unterscheidet Polyblends; denen die elastomeren Phasen physikalisch eingemischt vorliegen und Block-Copolymere in denen die elastomeren Phasen Bestandteil des polymeren Gerüsts sind. Durch den Aufbau der thermoplastischen Elastomere liegen harte und weiche Bereiche nebeneinander vor. Die harten Bereiche bilden dabei eine kristalline Netzstruktur oder eine kontinuierliche Phase deren Zwischenräume von elastomeren Segmenten ausgefüllt sind. Aufgrund dieses Aufbaus haben diese Werkstoffe kautschukähnliche Eigenschaften.

Bevorzugt kommen 3 Hauptgruppen der thermoplastischen Elastomere in Frage:
1. Copolyester
2. Polyether-Block-Amide (PEBA)
3. Thermoplastische Polyurethane (TPU)

Verfahren zur Synthese derartiger Copolyester sind aus DE-OS 22 39 271, DE-OS 22 13 128, DE-OS 24 49 343 und US-P 3,023,192 bekannt. Geeignete Copolyester im Sinne der Erfindung sind z. B. auf der Basis von Terephthalsäure mit gewissen Anteilen von Isophthalsäure sowie Butandiol und Polyethern, bevorzugt C₄-Polyethern auf der Basis von Tetrahydofuran und beispielsweise unter den Handelsnamen Hytrel der Fa. Du Pont, Pelpren der Fa. Toyobo, Arnitel der Fa. Akzo oder Ectel der Fa. Eastman Kodak erhältlich.

Verfahren zur Synthese der PEBA-Polymere sind aus FR-P 7 418 913 (Nr. der Veröffentlichung 2 273 021), DE-OS 28 02 989, DE-OS 28 37 687, DE-OS 25 23 991, EP 0 095 893 B2, DE-OS 27 12 987 beziehungsweise DOS 27 16 004 bekannt. Erfindungsgemäß bevorzugt geeignet sind solche PEBA-Polymere, die im Gegensatz zu den vorher beschriebenen, statistisch aufgebaut sind. Bausteine sind beispielsweise Adipinsäure, Aminododecansäure, anteilig Hexamethylendiamin, Polytetrahydrofuran, anteilig Polyethylenglykol.

Die erfindungsgemäß einsetzbaren thermoplastisch verarbeitbaren. Polyurethane sind durch Umsetzung der polyurethanbildenden Komponenten
A) organisches Diisocyanat,
B) lineares hydroxylterminiertes Polyol mit einem Molekulargewicht von 500 bis 10000,
C) Kettenverlängerer mit einem Molekulargewicht von 60 bis 500,
wobei das Molverhältnis der NCO-Gruppen in A) zu den gegenüber Isocyanat reaktiven Gruppen in B) u. C) 0,9 bis 1,2 beträgt, erhältlich.

Als organische Diisocyanate A) kommen beispielsweise aliphatische, cycloaliphatische, heterocyclische und aromatische Diisocyanate in Betracht, wie sie in Justus Liebigs Annalen der Chemie, 562, S. 75-136 beschrieben werden. Bevorzugt sind aliphatische und cycloaliphatische Diisocyanate.

Im einzelnen seien beispielhaft genannt: aliphatische Diisocyanate, wie Hexamethylendiisocyanat, cycloaliphatische Diisocyanate, wie Isophorondiisocyanat, 1,4-Cyclohexan-diisocyanat, 1-Methyl-2,4-cyclohexan-diisocyanat und 1-Methyl-2,6-cyclohexan-diisocyanat sowie die entsprechenden Isomerengemische, 4,4'-Dicyclohexylmethan-diisocyanat, 2,4'-Dicyclohexylmethan-diisocyanat und 2,2'-Dicyclohexylmethan-diisocyanat sowie die entsprechenden Isomerengemische, aromatische Diisocyanate, wie 2,4-Toluylendiisocyanat Gemische aus 2,4-Toluylendiisocyanat und 2,6-Toluylendiisocyanat, 4,4'-Diphenylmethandiisocyanat, 2,4'-Diphenylmethandiisocyanat und 2,2'-Diphenylmethandiisocyanat, Gemische aus 2,4'-Diphenylmethandiisocyanat und 4,4'- Diphenylmethandiisocyanat, Urethan, modifizierte flüssige 4,4'-Diphenylmethandiisocyanate und 2,4'-Diphenylmethandiisocyanate, 4,4'-Diisocyanatodiphenyl-ethan-(1,2) und 1,5-Naphthylendiisocyanat. Vorzugsweise verwendet werden 1,6-Hexamethylendiisocyanat, Isophorondiisocyanat, Dicyclohexylmethandiisocyanat, Diphenylmethandiisocyanat-Isomerengemische mit einem 4,4'- Diphenylmethandiisocyanatgehalt von >96 Gew.-% und insbesondere 4,4'-Diphenylmethandiisocyanat und 1,5-Naphthylendiisocyanat. Die genannten Diisocyanate können einzeln oder in Form von Mischungen untereinander zur Anwendung kommen. Sie können auch zusammen mit bis zu 15 Gew.-% (berechnet auf die Gesamtmenge an Diisocyanat) eines Polyisocyanates verwendet werden, beispielsweise Triphenylmethan-4,4',4"-triisocyanat oder Polyphenyl-polymethylen-polysocyanaten.

Als Komponente B) werden lineare hydroxylterminierte Polyole mit einem mittleren Molekulargewicht *Mn* von 500 bis 10000, bevorzugt 500 bis 5000, besonders bevorzugt 600 bis 2000 eingesetzt. Produktionsbedingt enthalten diese oft kleine Mengen an verzweigten Verbindungen. Häufig spricht man daher auch von "im wesentlichen linearen Polyolen". Bevorzugt sind Polyether-Diole, Polycarbonat-Diole, sterisch gehinderte Polyesterdiole, hydroxylterminierte Polybutadiene oder Gemische aus diesen.

Als Weichsegmente können allein oder im Gemisch mit den oben genannten Diolen auch Polysiloxan-Diole der Formel (IV)

HO-(CH₂)ₙ-[Si(R¹)₂-O-]ₘSi(R¹)₂-(CH₂)ₙ-OH (IV)

in welcher
- R¹: für eine Alkyl-Gruppe mit 1 bis 6 C-Atomen oder eine Phenyl-Gruppe steht,
- m: für 1 bis 30, bevorzugt 10 bis 25 und besonders bevorzugt 15 bis 25 steht, und
- n: für 3 bis 6 steht,
eingesetzt werden. Es sind bekannte Produkte und können nach an sich bekannten Synthesemethoden hergestellt werden, beispielsweise durch Umsetzung eines Silans der Formel (V)

H-[Si(R¹)₂-O-]ₘSi(R¹)₂-H (V)

in welcher R¹ und m die oben angegebenen Bedeutungen haben,
im Verhältnis 1 : 2 mit einem ungesättigten, aliphatischen oder cycloaliphatischen Alkohol wie Allylalkohol, Buten-(1)-ol oder Penten-(1)-ol in Gegenwart eines Katalysators, z. B. Hexachloroplatinsäure.

Geeignete Polyether-Diole können dadurch hergestellt werden, dass man ein oder mehrere Alkylenoxide mit 2 bis 4 Kohlenstoffatomen im Alkylenrest mit einem Startermolekül, das zwei aktive Wasserstoffatome gebunden enthält, umsetzt. Als Alkylenoxide seien z. B. genannt:

Ethylenoxid, 1,2-Propylenoxid, Epichlorhydrin und 1,2-Butylenoxid und 2,3-Butylenoxid. Vorzugsweise werden Ethylenoxid, Propylenoxid und Mischungen aus 1,2-Propylenoxid und Ethylenoxid eingesetzt. Die Alkylenoxide können einzeln, alternierend nacheinander oder als Mischungen verwendet werden. Als Startermoleküle kommen beispielsweise in Betracht: Wasser, Aminoalkohole, wie N-Alkyl-diethanolamine, beispielsweise N-Methyl-diethanol-amin, und Diole, wie Ethylenglykol, 1,3-Propylenglykol, 1,4-Butandiol und 1,6-Hexandiol. Gegebenenfalls können auch Mischungen von Startermolekülen eingesetzt werden. Geeignete Polyether-Diole sind ferner die hydroxylgruppen-haltigen Polymerisationsprodukte des Tetrahydrofurans. Es können auch trifunktiorielle Polyether in Anteilen von 0 bis 30 Gew.-%, bezogen auf die bifunktionellen Polyether, eingesetzt werden, jedoch höchsten in solcher Menge, dass ein thermoplastisch verarbeitbares Produkt entsteht. Die im wesentlichen linearen Polyether-Diole können sowohl einzeln als auch in Form von Mischungen untereinander zur Anwendung kommen.

Geeignete sterisch gehinderte Polyester-Diole können beispielsweise aus Dicarbonsäuren mit 2 bis 12 Kohlenstoffatomen, vorzugsweise 4 bis 6 Kohlenstoffatomen, und mehrwertigen Alkoholen hergestellt werden. Als Dicarbonsäuren kommen beispielsweise in Betracht: aliphatische Dicarbonsäuren, wie Bernsteinsäure, Glutarsäure, Adipinsäure, Korksäure, Azelainsäure und Sebacinsäure und aromatische Dicarbonsäuren, wie Phthalsäure, Isophthalsäure und Terephthalsäure. Die Dicarbonsäuren können einzeln oder als Gemische, z. B. in Form einer Bernstein-, Glutar- und Adipinsäuremischung, verwendet werden. Zur Herstellung der Polyester-Diole kann es gegebenenfalls vorteilhaft sein, anstelle der Dicarbonsäuren die entsprechenden Dicarbonsäurederivate, wie Carbonsäurediester mit 1 bis 4 Kohlenstoffatomen im Alkoholrest, Carbonsäureanhydride oder Carbonsäurechloride zu verwenden. Beispiele für mehrwertige Alkohole sind sterisch gehinderte Glykole mit 2 bis 10, vorzugsweise 2 bis 6 Kohlenstoffatomen, die in beta-Position zur Hydroxylgruppe mindestens einen Alkylrest tragen, wie 2,2-Dimethyl-1,3-propandiol, 2-Methyl-2-propyl-1,3-propandiol, 2,2-Diethyl-1,3-propandiol, 2-Ethyl-1,3-hexandiol, 2,5-Dimethyl-2,5- hexandiol, 2,2,4-Trimethyl-1,3-pentandiol, oder Gemische mit Ethylenglykol, Diethylenglykol, 1,4-Butandiol, 1,5-pentandiol, 1,6-Hexandiol, 1,10-Decandiol, 1,3-Propandiol und Dipropylenglykol. Je nach den gewünschten Eigenschaften können die mehrwertigen Alkohole allein oder gegebenenfalls in Mischung untereinander verwendet werden. Geeignet sind ferner Ester der Kohlensäure mit den genannten Diolen, insbesondere solchen mit 3 bis 6 Kohlenstoffatomen, wie 2,2-Dimethyl-1,3-propandiol oder 1,6-Hexandiol, Kondensationsprodukte von Hydroxycarbonsäuren, beispielsweise Hydroxycapronsäure und Polymerisationsprodukte von Lactonen, beispielsweise gegebenenfalls substituierten Caprolactonen. Als Polyester-Diole werden vorzugsweise verwendet, Neopentylglykol-polyadipate 1,6-Hexandiol-neopentylglykol-polyadipate. Die Polyester-Diole können einzeln oder in Form von Mischungen untereinander zur Anwendung kommen.

Gegebenenfalls können neben Polyesterdiolen andere Polyole eingesetzt werden zum Beispiel Polycarbonatdiole, Polyetherdiole und Gemische daraus.

Als Hydroxylgruppen aufweisende Polycarbonate kommen solche der an sich bekannten Art in Betracht, die z. B. durch Umsetzung von Diolen wie Propandiol-(1,3), Butandiol-(1,4) und/oder Hexandiol-(1,6), Diethylenglykol, Triethylenglykol, Tetraethylenglykol oder Thiodiglykol mit Diarylcarbonaten, z. B. Diphenylcarbonat oder Phosgen hergestellt werden können (DE-AS 16 94 080, DE-OS 22 21 751).

Neben den Polyesterpolyolen und den Polycarbonatdiolen können auch Gemische aus Polyetherpolyolen und Polyesterpolyolen und Gemische aus Polyetherpolyolen Polycarbonatdiolen mit jeweils einem zahlenmittleren Molekulargewicht zwischen 600 und 5000 g/mol, bevorzugt 700 und 4200 g/mol eingesetzt werden.

Als Kettenverlängerungsmittel C) werden Diole, Diamine oder Aminoalkohole mit einem Molekulargewicht von 60 bis 500 eingesetzt, vorzugsweise aliphatische Diole mit 2 bis 14 Kohlenstoffatomen, wie z. B. Ethandiol, 1,6-Hexandiol, Diethylenglykol, Dipropylenglykol und insbesondere 1,4-Butandiol. Geeignet sind jedoch auch Diester der Terephthalsäure mit Glykolen mit 2 bis 4 Kohlenstoffatomen, wie z. B. Terephthalsäure-bis-ethylenglykol oder Terephthalsäure- bis-1,4-butandiol, Hydroxyalkylenether des Hydrochinons, wie z. B. 1,4-Di(-hydroxyethyl)-hydrochinon, ethoxylierte Bisphenole, (cyclo)aliphatische Diamine, wie z. B. Isophorondiamin, Ethylendiamin, 1,2-Propylen-diamin, 1,3-Propylen-diamin, N-Methyl-propylen-1,3-diamin, 1,6-Hexamethylendiamin, 1,4-Diaminocyclohexan, 1,3-Diaminocyclohexan, N,N'-Dimethyl-ethylen-diamin und 4,4'-Dicyclohexylmethandiamin und aromatische Diamine, wie z. B. 2,4-Toluylendiamin und 2,6-Toluylen-diamin, 3,5-Diethyl-2,4-toluylen-diamin und 3,5-Diethyl-2,6-toluylendiamin und primäre mono-, di-, tri- oder tetraalkylsubstituierte 4,4'-Diaminodiphenylmethane oder Aminoalkohole wie Ethanolamin, 1-Aminopropanol, 2-Aminopropanol. Es können auch Gemische der oben genannten Kettenverlängerer eingesetzt werden. Daneben können auch kleinere Mengen an tri- oder höherfunktionellen Vernetzern zugesetzt werden, z. B. Glycerin, Trimethylolpropan, Pentaerythrit, Sorbit. Besonders bevorzugt werden 1,4-Butandiol, 1,6-Hexandiol, Isophorondiamin und deren Gemische eingesetzt.

Weiterhin können in geringen Mengen auch übliche monofunktionelle Verbindungen eingesetzt werden, z. B. als Kettenabbrecher oder Entformungshilfen. Beispielhaft genannt seien Alkohole wie Octanol und Stearylalkohol oder Amine wie Butylamin und Stearylamin.

Die molaren Verhältnisse der Aufbaukomponenten können über einen breiten Bereich variiert werden, wodurch sich die Eigenschaften des Produkts einstellen lassen. Bewährt haben sich molare Verhältnisse von Polyolen zu Kettenverlängerern von 1 : 1 bis 1 : 12. Das Molverhältnis von Diisocyanaten und Polyolen beträgt bevorzugt 1,2 : 1 bis 30 : 1. Besonders bevorzugt sind Verhältnisse von 2 : 1 bis 12 : 1. Zur Herstellung der TPU können die Aufbaukomponenten, gegebenenfalls in Gegenwart von Katalysatoren, Hilfsmitteln und Zusatzstoffen, in solchen Mengen zur Reaktion gebracht werden, dass das Äquivalenzverhältnis von NCO-Gruppen zur Summe der NCO reaktiven Gruppen, insbesondere der Hydroxy- oder Aminogruppen der niedermolekularen Diole/Triole, Amine und der Polyole 0,9 : 1 bis 1,2 : 1, vorzugsweise 0,98 : 1 bis 1,05 : 1, besonders bevorzugt 1,005 : 1 bis 1,01 : 1 beträgt.

Die erfindungsgemäß verwendbaren Polyurethane können ohne Katalysatoren hergestellt werden; in manchen Fällen kann der Einsatz von Katalysatoren jedoch angezeigt sein. Im allgemeinen werden die Katalysatoren in Mengen von bis zu 100 ppm, bezogen auf die Gesamtmenge an Edukten, verwendet. Geeignete erfindungsgemäße Katalysatoren sind die nach dem Stand der Technik bekannten und üblichen tertiären Amine, wie z. B. Triethylamin, Dimethylcyclohexylamin, N-Methylmorpholin, N,N'-Dimethyl-piperazin, 2-(Dimethylamino-ethoxy)-ethanol, Diazabicyclo[2,2,2]octan und ähnliche sowie insbesondere organische Metallverbindungen wie Titansäureester, Eisenverbindungen, Zinnverbindungen, z. B. Zinndiacetat, Zinndioctoat, Zinndilaurat oder die Zinndialkylsalze aliphatischer Carbonsäuren. Bevorzugt sind Dibutylzinndiacetat und Dibutylzinndilaurat; von diesen genügen Mengen von 1 bis 10 ppm, um die Reaktion zu katalysieren.

Neben den TPU-Komponenten und den Katalysatoren können auch andere Hilfsmittel und Zusatzstoffe zugesetzt werden. Genannt seien beispielsweise Gleitmittel wie Fettsäureester, deren Metallseifen, Fettsäureamide und Siliconverbindungen, Antiblockmittel, Inhibitoren, Stabilisatoren gegen Hydrolyse, Licht, Hitze und Verfärbung, Flammschutzmittel, Farbstoffe, Pigmente, anorganische oder organische Füllstoffe und Verstärkungsmittel. Verstärkungsmittel sind insbesondere faserartige Verstärkungsstoffe wie anorganische Fasern, die nach dem Stand der Technik hergestellt werden und auch mit einer Schlichte beaufschlagt sein können. Nähere Angaben über die genannten Hilfs- und Zusatzstoffe sind der Fachliteratur zu entnehmen, beispielsweise J. H. Saunders, K. C. Frisch: "High Polymers", Band XVI, Polyurethane, Teil 1 und 2, Interscience Publishers 1962 bzw. 1964, R. Gächter, H. Müller (Ed.): Taschenbuch der Kunststoff-Additive, 3. Ausgabe, Hanser Verlag, München 1989, oder DE-OS 29 01 774.

Der Aufbau der thermoplastisch verarbeitbaren Polyurethanelastomeren erfolgt bevorzugt schrittweise im so genannten Prepolymerverfahren. Beim Prepolymerverfahren wird aus dem Polyol und dem Diisocyanat ein isocyanathaltiges Prepolymer gebildet, das in einem zweiten Schritt mit dem Kettenverlängerer umgesetzt wird. Die TPU können kontinuierlich oder diskontinuierlich hergestellt werden. Die bekanntesten technischen Herstellverfahren sind das Bandverfahren und das Extruderverfahren.

Das Kunststoffpulver hat einen mittleren Teilchendurchmesser d₅₀ von 50 bis 800 µm, bevorzugt von 100 bis 500 µm.

Zur Einstellung dieses mittleren Teilchendurchmessers wird der Kunststoff beispielsweise gemahlen. Dafür kommen verschiedene bekannte Mühlen in Frage. Feinprallmühle, Spiralstrahlmühle, Fließbettgegenstrahlmühle, Schwingmühle. Bei weichen, zäh-elastische Kunststoffen kann die Mahlung bei Temperaturen unterhalb von Raumtemperatur erfolgen.

Für das erfindungsgemäße Verfahren wird der Wirkstoff-Pulver mit dem Kunststoff-Pulver zu einer Pulvermischung gemischt.

Über eine geeignete Dosiereinrichtung, K-Tron & Soder-Schnecke, oder Differential-Dosierwaage, wird die erhaltene Pulvermischung in die Öffnung des Extruders gefahren. Je nach Temperaturführung und Mischungsverhältnis kann das Mischungsverhältnis breit variiert werden. So können Pulvermischungen mit einem Gewichtsverhältnis von Kunststoff- zu Wirkstoff-Pulver von 95:1 bis 5:95, bevorzugt von 90:10 bis 10:95 eingesetzt werden.

Sofern erwünscht, kann zusätzlich zu der Pulvermischung weiterer Kunststoff extrudiert werden. Vorzugsweise ist der weitere Kunststoff der gleiche, der für die Herstellung der Pulvermischung eingesetzt wurde. Es kann auch ein wirkstoffhaltiger Masterbatch direkt mit dem Kunststoff verschmolzen werden oder mit der bereits vorliegenden Kunststoffschmelze gemischt werden.

Die Vermischung/Homogenisierung der Wirkstoff-Polymer-Pulver-Mischung mit dein Polymer kann nach bekannten Techniken über Kneter oder Schneckenmaschinen erfolgen, vorzugsweise in Ein oder Doppelschneckenextrudem in einem Temperaturbereich zwischen 150 und 200°C. Durch das Vermischen der Komponenten während des Extrusionsprozesses, wird eine homogene, molekulardisperse Verteilung des Wirkstoffs in der Polymermatrix erzielt, ohne dass zusätzliche Arbeitsschritte erforderlich wären.

Die Wirkstoffkonzentration der so erhaltenen Kunststoffzusammensetzung kann vorzugsweise 0,1 bis 5 Gew.-% betragen. Die Kunststoffzusammensetzung eignet sich für die Verwendung für die Herstellung von Kathetern und anderer medizintechnischer Produkte.

Die nachfolgenden Beispiele sollen die Erfindung illustrieren ohne sie jedoch zu beschränken.

### Beispiele

### Beispiel 1 (Vergleichsbeispiel)

Handelsübliches aromatisches Polyetherurethan mit 20 Gew.-% Bariumsulfat: Tecothane TT 2085 A-B20 der Shore-Härte 85 A (Fa. Noveon, Wobum MA) wurde als wirkstofffreies Zylindergranulat in Form von handelsüblichem Linsengranulat mit einer Größe von ca. 2 mm auf einem Zweiwellenextruder ZSK extrudiert. Der Wirkstoff Ciprofloxacin-Hydrochlorid (d₅₀ = 9,13 µm) wurde in das Gehäuse 1 des Zwei-Wellen-Extruders der Fa. Brabender mittels eine zwei-welligen Differential-Dosier-Waage als reines Pulver zudosiert. Es wurde eine Schmelze erhalten, die nach Abkühlung im Wasser/Luftbad und Stranggranulation ein farbloses, leicht trübes Zylindergranulat mit 1 Gew.-% Ciprofloxacin Hydrochlorid ergab.

Für die Bestimmung des Freisetzungsprofils des eingearbeiteten Wirkstoffs wurden Strangproben (2 mm Durchmesser und ca. 17 cm lang) genommen und das Granulat zu Prüfkörpern (Platten) spritzgegossen.

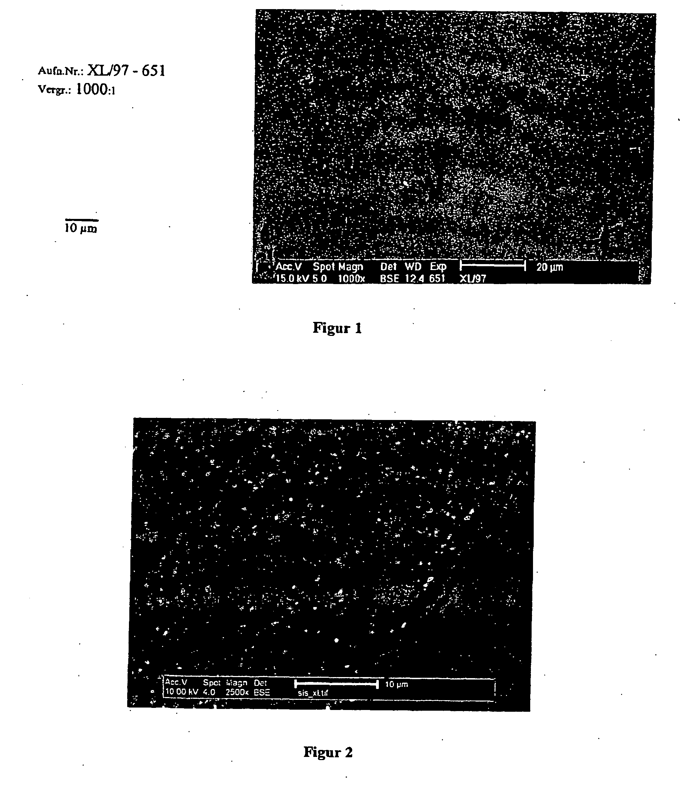

Zur Untersuchung der Verteilung des Wirkstoffs im Polymer wurden Rasterelektronenmikroskopische Aufnahmen der Oberfläche eines Zylinder-Granulat-Korns angefertigt (Figur 1). Die inhomogene Verteilung der Wirkstoff-Teilchen in der Matrix wird deutlich.

### Beispiel 2

Handelsübliches Tecothane TT2085A-B20 Linsengranulat mit einer Größe von ca. 2 mm wurde bei -40°C zu einem Pulver vermahlen, das anschließend in zwei Fraktionen gesiebt wurde. Eine 1. Fraktion mit d₅₀ = 300 µm wurde für die erfindungsgemäßen Beispiele verwendet, eine 2. Fraktion > 500µm wurde nicht verwendet

10 g Ciprofloxacin-Hydrochlorid (d₅₀ = 9,13 µm) wurden in einem Intensivmischer mit 990 g wirkstofffreies Tecothane TT2085A-B20-Pulver (d₅₀ = 300 µm) vermischt. Die Polymer-Wirkstoff-Pulvermischung wurde in Gehäuse 1 des Extruders dosiert. Das wirkstoffhaltige Zylindergranulat wurde auf einem Zweiwellenextruder ZSK der Fa. Brabender extrudiert. Es wurde eine klare Schmelze erhalten, die nach Abkühlung im Wasser/Luftbad und Stranggranulation ein farbloses, klares Zylindergranulat mit 1 Gew.-% Ciprofloxacin Hydrochlorid ergab.

Für die Bestimmung des Freisetzungsprofils des eingearbeiteten Wirkstoffs wurden Strangproben (2 mm Durchmesser und ca. 17 cm lang) genommen und das Granulat zu Prüfkörpern (Platten) spritzgegossen.

Zur Untersuchung der Verteilung des Wirkstoffs im Polymer wurden Rasterelektronenmikroskopische Aufnahmen der Oberfläche eines Zylinder-Granulat-Koms angefertigt (Figur 2). Die homogene Verteilung der Wirkstoff-Teilchen in der Matrix wird deutlich.

### Beispiel 3

10 g Octenidin Dihydrochlorid-Pulver (d₅₀ = 13,4 µm) wurde in einem Intensivmischer mit 990 g wirkstofffreies Tecothane TT2085A-B20-Pulver(d₅₀ = 300 µm) vermischt. Das wirkstoffhaltige Zylindergranulat wurde auf einem Zweiwellenextruder ZSK extrudiert. Es wurde eine klare Schmelze erhalten, die nach Abkühlung im Wasser/Luftbad und Stranggranulation ein farbloses, klares Zylindergranulat mit 1 Gew.-% Octenidin Dihydrochlorid ergab.

Für die Bestimmung des Freisetzungsprofils des eingearbeiteten Wirkstoffs durch Elutionsversuche wurden Strangproben (2 mm Durchmesser und ca. 17 cm lang) genommen und das Granulat zu Prüfkörpern (Platten) spritzgegossen.

Die Elutionsversuche wurden an Spritzgussplatten, die in 1 cm² große Stücke zerschnitten wurden, durchgeführt. Die Proben wogen jeweils ca. 2,2 g und hatten eine Oberfläche von 20,5 cm². Als Elutionsmedium wurden 16 ml demineralisiertes Wasser verwendet Nach 1 h, 4 h, 8 h, 24 h, 48 h, 120 h und 360 Stunden (15 Tage) wurde das Elutionsmedium Wasser jeweils gegen neues ausgetauscht und der Wirkstoff-Gehalt in den Lösungen bestimmt.

**Tabelle 1: Eluierte Wirkstoff-Menge bezogen auf die ursprüngliche vorhandene Menge**

| **Stunden** | Beispiel 1 (nicht erfindungsgemäß) | Beispiel 2 | Beispiel 3 |
|---|---|---|---|
| **1,00** | 0,111 % | 0,096% | 0,116% |
| **4,00** | 0,125% | 0,125% | 0,225% |
| **8,00** | 0,133% | 0,162% | 0,302% |
| **24,00** | 0,147% | 0,214% | 0,524% |
| **48,00** | 0,221% | 0,265% | 0,785% |
| **120,00** | 0,236% | 0,390% | 1,502% |
| **360,00** | 0,302% | 0,714% | 3,295% |

Summiert über alle 7 Lösungen waren nach 15 Tagen aus den Plättchen des nicht erfindungsgemäßen Beispiels 1 0,302%, aus den des Beispiels 2 0,714% und aus den Plättchen des Beispiels 3 3,295% der ursprünglichen Wirkstoffmenge extrahiert worden. Nach bereits 48 Stunden ist beim nicht erfindungsgemäßen Beispiel 1 die Nachdiffusion von Wirkstoff aus dem Bulk an die Oberfläche fast zum Erliegen gekommen. Durch die Agglomaration der Wirkstoff-Partikel haben sich - wie die REM-Aufnahme zeigt - "Wirkstoff-Nester" gebildet, aus denen der Wirkstofftransport an die Grenzschicht zum flüssigen Medium stark behindert ist.

## Patentansprüche

1. Verfahren zur Herstellung einer Kunststoffzusammensetzung enthaltend einen Kunststoff und einen Wirkstoff aus der Gruppe der Bis-(4-substituierten-amino-1-pyridinium)-alkan-Salze und deren Basen umfassend die Schritte
a) Herstellen einer Pulvermischung durch Vermischens eines Wirkstoffpulvers mit einem Kunststoffpulver mit einem mittleren Teilchendurchmesser d₅₀ von 50 bis 800 µm und
b) Extrudieren besagter Pulvermischung, gegebenenfalls mit weiterem Kunststoff.

2. Verfahren gemäß Anspruch 1, **dadurch gekennzeichnet, dass** das Gewichtsverhältnis von Kunststoffpulver zu Wirkstoffpulver im Schritt a) von 95:1 bis 5:95 beträgt.

3. Verfahren gemäß Anspruch 1 oder 2, **dadurch gekennzeichnet, dass** die erhaltene Kunststoffzusammensetzung eine Wirkstoffkonzentration von 0,1 bis 5 Gew.-% aufweist.

4. Verfahren gemäß einem der vorstehenden Ansprüche , **dadurch gekennzeichnet, dass** das Wirkstoffpulver eine mittlere Teilchengröße d₅₀ von 0,5 bis 20 µm aufweist.

5. Verfahren gemäß einem der vorstehenden Ansprüche, **dadurch gekennzeichnet, dass** der Kunststoff ein thermoplastisches Elastomer ist.

6. Verfahren gemäß Anspruch 5, **dadurch gekennzeichnet, dass** das thermoplastische Elastomer ausgewählt ist aus der Gruppe bestehend aus Copolyester, Polyether-Block-Amiden und Thermoplastische Polyurethanen.

7. Verfahren gemäß einem der vorstehenden Ansprüche, **dadurch gekennzeichnet, dass** der Wirkstoff ausgewählt ist aus der Gruppe bestehend aus Stoffen der allgemeinen Formeln (I) und (II) 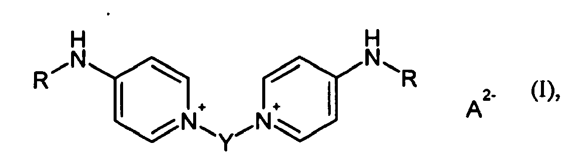 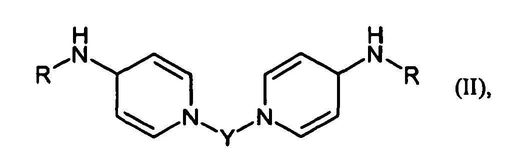 in welchen
Y für eine Alkylengruppe mit 4 bis 18 Kohlenstoffatomen steht,
R für C₆-C₁₈-Alkyl, C₅-C₇-Cycloalkyl oder durch ein Halogenatom substituiertes Phenyl steht und
A für zwei einwertige oder ein zweiwertiges Anion steht.

8. Verfahren gemäß einem der vorstehenden Ansprüche, **dadurch gekennzeichnet, dass** der Kunststoff ein thermoplastischem Polyurethan und der Wirkstoff Octenidin Dihydrochlorid ist.

9. Formkörper enthaltend eine Kunststoffzusammensetzung erhältlich nach einem der Ansprüche 1 bis 8.

10. Verwendung von Formkörpern gemäß Anspruch 9 für die Herstellung von Kathetern und anderen medizintechnischen Produkten.

## Claims

1. Process for preparation of a plastics composition comprising a plastic and comprising an active ingredient from the group of the bis(4-(substituted amino)-1-pyridinium)alkane salts and bases thereof encompassing the following steps
a) preparation of a powder mixture via mixing of an active ingredient powder with a plastics powder whose median particle diameter d₅₀ is from 50 to 800 µm and
b) extrusion of the said powder mixture, if appropriate with further plastic.

2. Process according to Claim 1, **characterized in that** the ratio by weight of plastics powder to active ingredient powder in step a) is from 95:1 to 5:95.

3. Process according to Claim 1 or 2, **characterized in that** the active ingredient concentration of the resultant plastics composition is from 0.1 to 5% by weight.

4. Process according to any of the preceding claims, **characterized in that** the median particle size d₅₀ of the active ingredient powder is from 0.5 to 20 *µ*m.

5. Process according to any of the preceding claims, **characterized in that** the plastic is a thermoplastic elastomer.

6. Process according to Claim 5, **characterized in that** the thermoplastic elastomer has been selected from the group consisting of copolyester, polyether block amides and thermoplastic polyurethanes.

7. Process according to any of the preceding claims, **characterized in that** the active ingredient has been selected from the group consisting of substances of the general formulae (I) and (II) 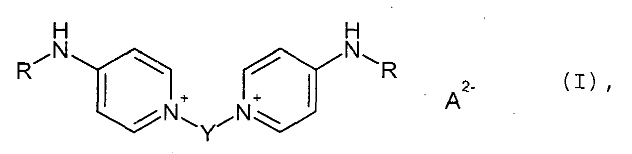 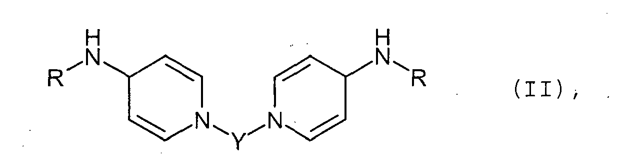 in which
Y is an alkylene group having from 4 to 18 carbon atoms,
R is C₆-C₁₈-alkyl, C₅-C₇-cycloalkyl or halogenatom-substituted phenyl and
A is two monovalent anions or one divalent anion.

8. Process according to any of the preceding claims, **characterized in that** the plastic is a thermoplastic polyurethane and the active ingredient is octenidine dihydrochloride.

9. Moulding comprising a plastics composition obtainable according to any of Claims 1 to 8.

10. Use of mouldings according to Claim 9 for production of catheters and of other medical-technology products.

## Revendications

1. Procédé de préparation d'une composition plastique contenant une matière plastique et un agent actif du groupe des sels de bis(4-(amino substitué)-1-pyridinium)alcane et leurs bases, comprenant les étapes de :
a) préparation d'un mélange pulvérulent par mélange d'une poudre d'agent actif à une poudre de matière plastique ayant un diamètre moyen des particules d₅₀ allant de 50 à 800 µm, et
b) extrusion du mélange pulvérulent, le cas échéant avec une autre matière plastique.

2. Procédé selon la revendication 1, **caractérisé en ce que** le rapport pondéral de la poudre plastique à la poudre d'agent actif à l'étape a) se situe dans l'intervalle allant de 95:1 à 5:95.

3. Procédé selon la revendication 1 ou 2, **caractérisé en ce que** la composition plastique obtenue présente une concentration en agent actif allant de 0,1 à 5% en poids.

4. Procédé selon l'une des revendications précédentes, **caractérisé en ce que** la poudre d'agent actif présente une taille moyenne des particules d₅₀ allant de 0,5 à 20 µm.

5. Procédé selon l'une des revendications précédentes, **caractérisé en ce que** la matière plastique est un élastomère thermoplastique.

6. Procédé selon la revendication 5, **caractérisé en ce que** l'élastomère thermoplastique est choisi parmi le groupe consistant en un copolyester, un polyéther avec des séquences amide et un polyuréthanne thermoplastique.

7. Procédé selon l'une des revendications précédentes, **caractérisé en ce que** l'agent actif est choisi parmi le groupe consistant en les substances des formules générales (I) et (II) : 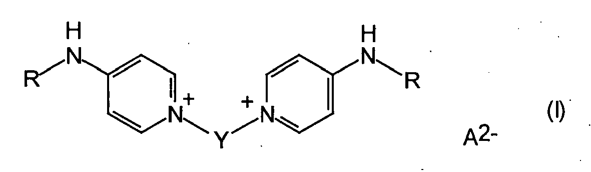 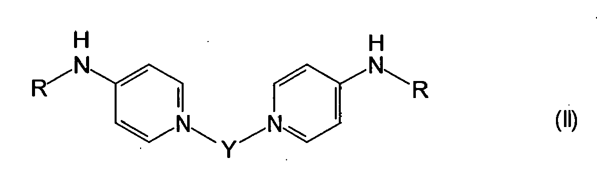 dans lesquelles
Y représente un radical alkylène ayant 4 à 18 atomes de carbone,
R représente un radical alcoyle en C₆-C₁₈, un radical cycloalcoyle en C₅-C₇ ou un radical phényle substitué par un atome d'halogène, et
A représente deux anions monovalents ou un anion bivalent.

8. Procédé selon l'une des revendications précédentes, **caractérisé en ce que** la matière plastique est un polyuréthanne thermoplastique et l'agent actif est le dichlorhydrate d'octénidine.

9. Corps moulé contenant une composition plastique pouvant être obtenue selon l'une des revendications 1 à 8.

10. Utilisation des corps moulés selon la revendication 9, pour la préparation de cathéters et autres produits de technique médicale.
